# EUROPEAN PATENT APPLICATION

(11) **EP 4 850 589 A1**
(43) Date of publication of application: **09.12.2026**
(21) Application number: 25803854.6
(22) Date of filing: 03.01.2025
(51) Int. Cl.: A61B 3/00, A61B 3/12, A61B 3/10, G06N 3/0475, G06N 3/094, G06N 3/088, G06N 3/096, G16H 50/50, G16H 30/20, G16H 30/40

(54) **HEALTH RISK PREDICTION APPARATUS AND METHOD BASED ON INTEGRATED LEARNING OF PLURALITY OF HEALTH INDEXES**

(30) Priority: 17.05.2024 KR 20240064709; 28.08.2024 KR 20240115609; 31.12.2024 KR 20240201942; 31.12.2024 KR 20240201943
(71) Applicant: Mediwhale Inc., Seoul 06049 (KR)
(72) Inventor: RIM, Hyung Taek, Seoul 06601 (KR); KIM, Hyeon Min, Seoul 06250 (KR); SEO, Chan Yang, Seoul 05318 (KR); NAM, Hyeon Seob, Seoul 06666 (KR)
(74) Representative: Nederlandsch Octrooibureau
(86) International application number: PCT/KR2025/000105
(87) International publication number: WO 2025/239496

(57) **Abstract**

The present disclosure relates to an apparatus and a method for predicting a health risk based on integrated learning of a plurality of health indicators. An apparatus for predicting a health risk based on integrated learning of a plurality of health indicators, for predicting a health risk of a subject, according to an embodiment, includes at least one processor, wherein the at least one processor is configured to: input a medical image of the subject to a first model that generates a plurality of predicted health indicators for predicting the health risk of the subject, so as to process the medical image; selectively extract some health indicators among the plurality of predicted health indicators; and analyze the health risk of the subject by inputting the selectively extracted health indicators to a second model that predicts the health risk of the subject, so as to process the selectively extracted health indicators.

## Description

### [Technical Field]

The present invention relates to the field of medical image processing and health risk prediction technology, and more particularly, to a model and a technique for predicting an individual's health condition and the risk of occurrence of a disease through a model that integrally learns a plurality of health indicators by utilizing data labeled based on clinical thresholds and that improves compatibility between medical images.

### [Background Art]

Recently, artificial intelligence (AI) technology utilizing medical image data enables early diagnosis of diseases and assessment of health risks, and has been recognized for its potential to contribute to preventive medical measures for various diseases such as cardiovascular disease, diabetes, and kidney disease.

However, the existing medical image-based AI technology has several limitations.

First, the ground truth labels used in the existing models were often generated in a manner that did not reflect clinical thresholds. Since the data labels are not directly linked to clinical judgment, this may act as a factor that lowers the clinical reliability of the prediction results generated by the AI model. For example, even though important health indicators such as the coronary artery calcium score (CAC score) or the kidney function indicator (eGFR) can be divided in their clinical meaning on the basis of specific thresholds, the existing technology used simple classification criteria that did not reflect such thresholds, thereby limiting the interpretability and effectiveness of the model as a result.

Second, there is a problem that data including both a medical image and a plurality of health indicators used as ground truth labels is insufficient. In practice, medical image data often does not comprehensively include various health indicators, and a specific indicator is frequently missing. For example, it is realistically difficult to secure data having, as labels, the coronary artery calcium score (CAC score), the kidney function indicator (eGFR), blood pressure, blood glucose level, and the like for a fundus image. Such a data shortage problem causes the AI model to be trained with limited training data, and as a result, the model performance is lowered and the reliability of the prediction result may be degraded.

Third, medical image data is affected by various factors such as the equipment manufacturer, the imaging environment, the resolution, and the quality, and due to such differences, a problem may occur in that the prediction results of the AI model are not consistent. In particular, the quality of the medical image and the difference between equipment cause a mismatch between the pattern learned by the AI model from the data and the actual data, thereby acting as a major cause of degrading the prediction performance.

Despite such limitations, the medical industry demands an AI-based diagnostic model capable of integrally considering a plurality of health indicators and reflecting the mutual relevance between diseases. In addition, there is a rapidly increasing demand for an AI technology that derives reliable results even in an environment where differences between various pieces of equipment exist, and that provides such results in a form that can be utilized in an actual medical field.

### [Disclosure]

### [Technical Problem]

The present invention is intended to solve the following two problems.

First, the present invention is intended to provide an AI-based prediction system that maximizes the utility of clinically meaningful data and can be effectively applied in an actual medical environment.

To this end, the present invention is intended to label a plurality of health indicators according to clinical thresholds so as to reflect the clinical meaning of each indicator, thereby increasing the clinical reliability of the model and maximizing the utility of the prediction results.

In addition, by adopting a scheme capable of simultaneously learning a plurality of health indicators related to a specific disease, or simultaneously learning a plurality of health indicators related to a plurality of diseases having high mutual relevance, the present invention is intended to integrally reflect the correlation between the indicators in the model, and thereby generate more precise and reliable prediction results. Furthermore, the present invention introduces a technique (self-training) for effectively utilizing partially missing health indicator data, so that medical image data can be maximally utilized even in a limited data environment.

Second, the present invention is intended to effectively overcome the compatibility problem caused by differences in quality and equipment between pieces of medical image data.

To this end, in order to minimize the difference between medical images generated by different equipment or imaging environments, the present invention is designed to measure the similarity between data by utilizing a distance-based pre-training method, and to learn in advance a feature extraction process reflecting the similarity.

In addition, the present invention is intended to provide a technique that introduces a statistics-based stabilization method to stabilize the data difference between medical images by adjusting clinical and statistical factors with respect to an existing simple deep learning model.

The problems to be solved by the present application are not limited to the above-mentioned problems, and problems not mentioned will be clearly understood by those of ordinary skill in the art to which the present invention pertains from the present specification and the accompanying drawings.

### [Technical Solution]

An apparatus for predicting a health risk based on integrated learning of a plurality of health indicators, for predicting a health risk of a subject, according to an embodiment, includes at least one processor, wherein the at least one processor is configured to: input a medical image of the subject to a first model that generates a plurality of predicted health indicators for predicting the health risk of the subject so as to process the medical image; selectively extract some health indicators among the plurality of predicted health indicators; and analyze the health risk of the subject by inputting the selectively extracted health indicators to a second model that predicts the health risk of the subject so as to process the selectively extracted health indicators, wherein the first model comprises a common sub-model that extracts an overall feature from the medical image to generate a feature vector for one or more tasks, and one or more head sub-models that receive the feature vector as an input to generate a prediction result for each health indicator, wherein the first model is trained simultaneously using a medical image and a plurality of health indicators as ground truth labels, and wherein the plurality of health indicators may be defined as labels classified by reflecting respective intrinsic clinical thresholds.

The plurality of health indicators may be composed of a plurality of binary labels classified by applying the respective intrinsic clinical thresholds.

The first model may be subjected to multitask learning for simultaneously predicting the plurality of predicted health indicators.

When a ground truth label for a predetermined health indicator among the plurality of health indicators does not exist at the time of training the first model, a predicted value of the predetermined health indicator may be generated through the first model, and the predicted value may be used as a predicted ground truth label of the predetermined health indicator so that the first model is additionally trained.

The second model may be a statistics-based model for analyzing the health risk of the subject based on the plurality of predicted health indicators.

The at least one processor may additionally input clinical data of the subject to the second model, and analyze the health risk of the subject by combining the selectively extracted health indicators and the clinical data of the subject.

The medical image may include a fundus image.

The health risk of the subject may include at least one of cerebro-cardiovascular disease, kidney disease, neurological disease, and liver disease.

In addition, a prediction apparatus that improves compatibility between medical images, for predicting a health risk of a subject, according to an embodiment, includes at least one processor, wherein the at least one processor is configured to: input a first fundus image captured under a first condition to a first model that generates a plurality of predicted health indicators for predicting the health risk of the subject so as to process the first fundus image; and analyze the health risk of the subject by using a second model that analyzes the health risk of the subject based on at least one of the plurality of predicted health indicators, wherein the first model comprises a common sub-model that extracts an overall feature from the first fundus image to generate a feature vector for one or more tasks, and one or more head sub-models that receive the feature vector as an input to generate a prediction result for each health indicator, wherein the first model is trained using a second fundus image and a plurality of health indicators as ground truth labels, wherein the second fundus image is captured under a second condition different from the first condition, and wherein the second model is a statistics-based model that analyzes the health risk of the subject in consideration of the plurality of predicted health indicators, and may stabilize the variability of the prediction result for each of the plurality of health indicators based on a statistical scheme.

The at least one processor may additionally input clinical data of the subject to the second model, and analyze the health risk of the subject by combining at least one of the plurality of predicted health indicators and the clinical data of the subject.

The clinical data may include age or gender.

The first model may be pre-trained through distance-based learning in which a plurality of fundus images obtained from the same person are configured as one positive pair.

The one positive pair may include a plurality of images captured under different conditions, wherein the different conditions may include at least one of a type of a camera that captured the fundus image, a field of view of the camera, whether a mydriatic agent has been applied, whether an imaging target is a left eye or a right eye, and an operator of the camera or an imaging environment.

The plurality of fundus images may include the second fundus image, and the first condition and the second condition may be included in the different conditions.

The plurality of health indicators may be defined as labels classified by reflecting respective intrinsic clinical thresholds.

The plurality of health indicators may be composed of a plurality of binary classification labels by applying different thresholds to the same health indicator.

The first model may be subjected to multitask learning for simultaneously predicting the plurality of predicted health indicators.

When a ground truth label for a predetermined health indicator among the plurality of health indicators does not exist at the time of training the first model, a predicted value of the predetermined health indicator may be generated through the first model, and the predicted value may be used as a predicted ground truth label of the predetermined health indicator so that the first model is additionally trained.

The health risk of the subject may include at least one of cerebro-cardiovascular disease, kidney disease, neurological disease, and liver disease.

The solving means of the problems of the present application are not limited to the above-mentioned solving means, and solving means not mentioned will be clearly understood by those of ordinary skill in the art to which the present invention pertains from the present specification and the accompanying drawings.

### [Advantageous Effects]

The present invention labels a plurality of health indicators according to clinical thresholds so as to reflect the clinical meaning of each indicator, and adopts a scheme capable of simultaneously learning a plurality of health indicators related to a specific disease, thereby integrally reflecting the correlation between the indicators in the model. In addition, by introducing a technique (self-training) for effectively utilizing partially missing health indicator data, the present invention can maximize the utility of clinically meaningful data and provide an AI-based prediction system that can be effectively applied in an actual medical environment.

In addition, based on the distance-based pre-training method, the present invention is designed to measure the similarity between data and to learn in advance a feature extraction process reflecting the similarity, thereby minimizing the difference between various medical images. Furthermore, by introducing a statistics-based stabilization method, the present invention adjusts clinical and statistical factors with respect to an existing simple deep learning model, thereby effectively correcting the difference between medical images generated by different equipment or imaging environments.

The effects of the present application are not limited to the above-mentioned effects, and effects not mentioned will be clearly understood by those of ordinary skill in the art to which the present invention pertains from the present specification and the accompanying drawings.

### [Description of Drawings]

FIG. 1 is a diagram for explaining a configuration of a diagnostic apparatus according to an embodiment.
FIG. 2 is a diagram for explaining a first model and a second model according to an embodiment.
FIG. 3 is a diagram for explaining multitask learning according to an embodiment.
FIG. 4 is a diagram for explaining self-training according to an embodiment.
FIG. 5 is a diagram for explaining different conditions of a fundus image according to an embodiment.
FIGS. 6 and 7 are diagrams for explaining prediction performance according to a statistics-based stabilization method according to an embodiment.
FIG. 8 is a diagram for explaining patient-level contrastive learning according to an embodiment.
FIG. 9 is a diagram for explaining prediction performance according to patient-level contrastive learning according to an embodiment.
FIG. 10 is a diagram for explaining an integrated compatibility model according to an embodiment.

### [Best Mode]

An apparatus for predicting a health risk based on integrated learning of a plurality of health indicators, for predicting a health risk of a subject, according to an embodiment, includes at least one processor, wherein the at least one processor is configured to: input a medical image of the subject to a first model that generates a plurality of predicted health indicators for predicting the health risk of the subject so as to process the medical image; selectively extract some health indicators among the plurality of predicted health indicators; and analyze the health risk of the subject by inputting the selectively extracted health indicators to a second model that predicts the health risk of the subject so as to process the selectively extracted health indicators, wherein the first model comprises a common sub-model that extracts an overall feature from the medical image to generate a feature vector for one or more tasks, and one or more head sub-models that receive the feature vector as an input to generate a prediction result for each health indicator, wherein the first model is trained simultaneously using a medical image and a plurality of health indicators as ground truth labels, and wherein the plurality of health indicators may be defined as labels classified by reflecting respective intrinsic clinical thresholds.

### [Mode for Invention]

The advantages and features of the present invention, and the methods of achieving them, will become apparent with reference to the embodiments described in detail below together with the accompanying drawings. However, the present invention is not limited to the embodiments disclosed below, but may be implemented in various different forms; rather, these embodiments are provided so that the disclosure of the present invention is complete and so as to fully inform those of ordinary skill in the art to which the present invention pertains of the scope of the present invention, and the present invention is defined only by the scope of the claims.

The terms used in the present specification are for describing the embodiments and are not intended to limit the present invention. In the present specification, the singular form also includes the plural form unless specifically stated otherwise in the phrase. As used in the specification, "comprises" and/or "comprising" do not exclude the presence or addition of one or more other components in addition to the mentioned components. The same reference numerals refer to the same components throughout the specification, and "and/or" includes each and every combination of one or more of the mentioned components. Although "first," "second," and the like are used to describe various components, these components are, of course, not limited by these terms. These terms are used only to distinguish one component from another component. Therefore, a first component mentioned below may, of course, be a second component within the technical idea of the present invention.

Unless otherwise defined, all terms (including technical and scientific terms) used in the present specification may be used with meanings that can be commonly understood by those of ordinary skill in the art to which the present invention pertains. In addition, terms defined in commonly used dictionaries are not interpreted in an idealized or excessive manner unless clearly and specifically defined.

In the present specification, an "image" may mean multi-dimensional data composed of discrete image elements (for example, pixels in a two-dimensional image and voxels in a three-dimensional image).

### Basic configuration: Training apparatus and diagnostic apparatus

Hereinafter, a diagnostic system that performs diagnosis of a subject by using medical image data will be described. In the present specification, the term "diagnosis" may mean diagnostic assistance for predicting a health risk, rather than directly diagnosing a disease. In addition, in the present specification, the term "diagnosis" may be used as the term "prediction" in that it predicts a health risk. For example, in the present specification, the diagnostic system may be expressed as a prediction system, and the diagnostic apparatus may be expressed as a prediction apparatus. Hereinafter, the term "diagnosis" is used for convenience of description, but this may mean diagnostic assistance and/or prediction.

In addition, the health risk may include at least one of cerebro-cardiovascular disease, kidney disease, neurological disease, liver disease, diabetes, hypertension, hypotension, and hyperlipidemia. In addition, the health risk may include various diseases that may occur in the subject.

The diagnostic system may include a training apparatus and a diagnostic apparatus. The training apparatus may perform training of a model. Here, the model may be at least one or more machine learning and/or deep learning models. For example, the training apparatus may train the model by performing preprocessing, training, validation, testing, and the like using the medical image data. In addition, the diagnostic apparatus may predict the health risk of the subject by using the model trained by the training apparatus.

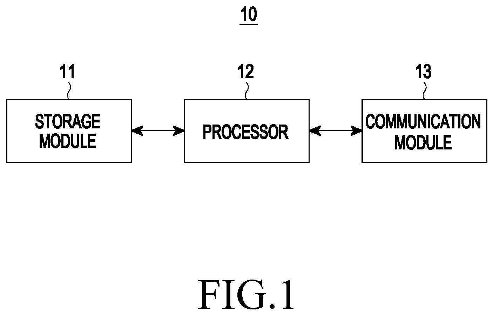

FIG. 1 is a diagram for explaining a configuration of a diagnostic apparatus according to an embodiment.

Referring to FIG. 1, the diagnostic apparatus may include a processor 12, a storage module 11, and a communication module 13.

The processor 12 may include one or more of a central processing unit (CPU), a random access memory (RAM), a graphics processing unit (GPU), one or more microprocessors, and other electronic components capable of processing input data according to predetermined logic. In addition, the processor 12 may be at least one or more.

The processor 12 may read a system program and various processing programs stored in the storage module 11. As an example, the processor 12 may deploy, on the RAM, a process, a method, and the like for performing the diagnosis described later, and may perform various types of processing according to the deployed program. For example, the processor 12 may process the algorithm described in the present specification. The processor 12 may predict the health risk by using a machine learning model. In addition, the processor 12 may implement various methods described in the present specification.

The storage module 11 may store the model. For example, the storage module 11 may store a machine learning model, parameters of the machine learning model, variables, the algorithm described in the present specification, and the like. In addition, the storage module 11 may store the model trained by the training apparatus.

The storage module 11 may be implemented as a nonvolatile semiconductor memory, a hard disk, a flash memory, a RAM, a read only memory (ROM), an electrically erasable programmable read-only memory (EEPROM), or other tangible nonvolatile recording medium, or the like.

The storage module 11 may store various processing programs, parameters for performing the processing of the programs, or such processing result data, and the like. As an example, the storage module 11 may store a program for performing the diagnosis described later, parameters, and data obtained according to the execution of such a program, and the like. In addition, the storage module 11 may store various machine learning models described later.

Although not shown, the diagnostic apparatus may further include an input module. The input module may obtain a user input. For example, the input module may obtain a user input. In addition, the input module may obtain the body information of the subject described in the present specification (at least one of height, weight, age, gender, race, smoking status, blood pressure (for example, blood pressure value, whether hypertension is present), whether diabetes is present (or blood glucose level), and cholesterol level), and the like.

The communication module 13 may communicate with various apparatuses (for example, the training apparatus). For example, the diagnostic apparatus may receive, from the training apparatus through the communication module 13, the model trained by the training apparatus. As an example, the communication module 13 may include a mobile communication module and/or a wired/wireless module that transmits data through various other communication standards.

In addition, the diagnostic apparatus may be implemented in the form of a server. In addition, the diagnostic apparatus may be implemented in the form of a client apparatus.

In addition, the training apparatus may also include a storage module, a processor, and a communication module. With respect to the storage module, the processor, and the communication module of the training apparatus, at least a part of the description of the above-described storage module 11, processor 12, and communication module 13 may be applied, and for convenience of description, the description of the storage module, the processor, and the communication module of the training apparatus is omitted.

### Basic configuration: Data, model structure, and operation scheme

The medical image data may include various types of medical images. As an example, the medical image data may include an ophthalmology-related medical image, a cardiovascular-related medical image, and a lung- and respiratory-related medical image.

For example, the ophthalmology-related medical image includes a fundus image, optical coherence tomography (OCT), and the like, and the cardiovascular-related medical image includes a cardiac CT, a cardiac MRI, a cardiac ultrasound, a carotid ultrasound, an intravascular ultrasound (IVUS), and the like. In addition, the lung- and respiratory-related medical image includes a chest X-ray and a chest CT, and in addition, the medical image data may include a brain CT, angiography, and the like.

A plurality of health indicators may be labeled in the medical image data, and this may be utilized to evaluate the current state of a patient related to a specific disease, or to predict the risk of occurrence of a specific disease in the future. Each of such a plurality of health indicators has a clinical meaning, and serves as an important criterion for analyzing the overall health condition of the patient from various angles.

The plurality of health indicators may include a cardiovascular health indicator, and the cardiovascular health indicator may be divided into a direct indicator and an indirect indicator. The direct indicator may include an imaging biomarker that reflects arteriosclerosis and general vessel status. Such indicators include the coronary artery calcium score (CAC score), the carotid intima-media thickness (CIMT), arterial plaque information, and the like. Such indicators may be sub-classified as a cardiovascular imaging biomarker.

The coronary artery calcium score is used to directly evaluate the degree of arteriosclerosis by measuring the degree of calcification in the coronary artery. The carotid intima-media thickness is an indicator that evaluates the arterial health condition by measuring the thickness of the carotid artery wall. In addition, the arterial plaque information determines the vessel condition by analyzing detailed characteristics such as the size, volume, composition, and stability of the plaque. Additionally, retinal vessel characteristics can also be used as a direct indicator. Characteristics such as vessel tortuosity, caliber, and fractal dimension observed in a retinal image provide important information related to cardiovascular health.

The indirect indicator is a non-imaging biomarker and is used to complementarily evaluate cardiovascular health. Such indicators include blood pressure, blood glucose level, cholesterol level, smoking status, HbA1c, age, gender, body mass index (BMI), and the like. Blood pressure indirectly evaluates the state of the cardiovascular system through systolic and diastolic blood pressure. The blood glucose level is utilized to check the presence of diabetes and the blood glucose control state through fasting blood glucose and HbA1c levels. The cholesterol level includes total cholesterol, LDL, HDL, and triglyceride levels, and plays an important role in indirectly evaluating cardiovascular risk. Smoking status is one of the cardiovascular disease risk factors, and current and past smoking habits are utilized as an indicator. In addition, age, gender, body mass index (BMI), family history of cardiovascular disease, and the like are also important indirect indicators for evaluating the cardiovascular health condition.

In addition, an eye disease may also provide a notable source of information in relation to cardiovascular disease. For example, diabetic retinopathy is an eye disease closely related to vessel health, and provides a clue capable of indirectly identifying systemic vessel health through the state of the retinal vessels in a diabetic patient. In addition, retinal vein occlusion, retinal artery occlusion, hypertensive retinopathy, macular degeneration, retinal microangiopathy, ischemic optic nerve lesions, retinal neovascularization, and the like provide information related to cardiovascular disease.

In addition, the plurality of health indicators together with the medical image data are utilized as training data of a health risk prediction model (machine learning model), and play an important role in evaluating the health condition of the patient with respect to a specific disease and predicting a future risk.

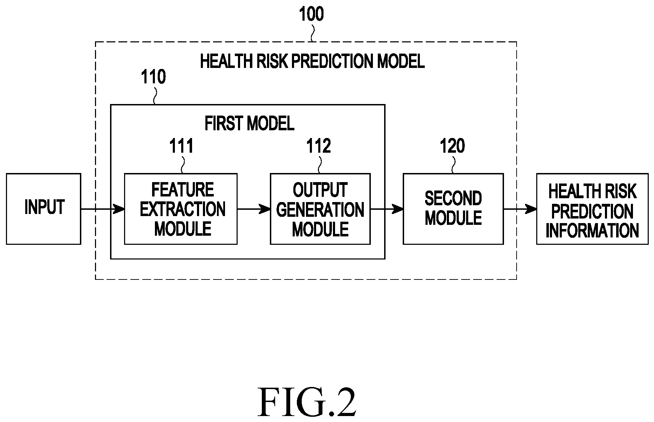

FIG. 2 is a diagram for explaining a health risk prediction model according to an embodiment.

Referring to FIG. 2, the processor of the diagnostic apparatus may input the medical image data to a health risk prediction model 100, and may process the medical image data by using the health risk prediction model 100 to generate health risk prediction information.

The health risk prediction model 100 may include a first model 110 for predicting, based on the medical image data, a plurality of health indicators for predicting the health risk of an individual, and a second model 120 for predicting the individual health risk by analyzing the plurality of health indicators generated thereby. The health risk prediction model 100 may be included in the diagnostic apparatus. For example, the health risk prediction model 100 may be stored in the storage module of the diagnostic apparatus, and the processor of the diagnostic apparatus may load the health risk prediction model 100 from the storage module and predict the health risk of the subject by using the health risk prediction model 100.

The first model 110 receives the medical image data as an input and generates a plurality of health indicators, and may be based on a neural network-based model. The second model 120 analyzes the output value of the first model, that is, the plurality of health indicators, to evaluate the health risk of the individual, and may be based on a statistics-based model.

Specifically, the first model 110 may be a neural network-based model that processes the medical image data to generate a plurality of health indicators. The processor may process the medical image data by using the first model 110 to generate a plurality of health indicators predicted with respect to the current health condition of the subject from which the medical image data has been scanned. The plurality of health indicators may include health indicators for a plurality of diseases having high mutual relevance, such as a cardiovascular health indicator (for example, CAC score) and a kidney health indicator (for example, eGFR). In addition, the processor may selectively extract a health indicator included in a specific disease among the plurality of health indicators.

Cardiovascular disease, kidney disease, diabetes, and obesity are diseases closely related to each other. These diseases share common pathological mechanisms of metabolic abnormality, inflammation, and decline of vessel and kidney function, and the onset and progression of each disease affect the other diseases. To systematically understand this, the American Heart Association (AHA) has presented the Cardiovascular-Kidney-Metabolic (CKM) syndrome model. This model describes the disease progression stages in a structured manner, and clarifies the interaction between cardiovascular disease, kidney disease, diabetes, and obesity. In particular, it shows the process in which, after the stage where metabolic risk factors and chronic kidney disease appear, the disease progresses to the stage where potential cardiovascular disease appears, and finally leads to the stage of overt clinical cardiovascular disease. As such, when a plurality of health indicators related to diseases having high mutual relevance are simultaneously learned, the mutual relevance between the diseases can be effectively reflected.

More specifically, the first model 110 may include a feature extraction module 111 and an output generation module 112.

First, the feature extraction module 111 may receive the medical image data as an input, and may extract a main feature of the input medical image data. In an embodiment, the feature extraction module 111 may be configured as a neural network-based common sub-model, and may automatically learn clinically meaningful information by processing the medical image data. First, the processor may convert the medical image data into a form suitable for processing in the feature extraction module 111 by performing operations such as pixel intensity adjustment, resolution change, and noise removal with respect to the medical image data through a preprocessing process. Thereafter, the feature extraction module 111 may detect important patterns such as a lesion pattern, a vessel structure, and a tissue density in the image by utilizing a deep learning technique such as a convolutional neural network (CNN), and may convert them into a data representation (feature vector). The generated feature vector is a data representation commonly usable among the plurality of health indicators, and may include important information necessary for prediction of each indicator. This process contributes to increasing the learning efficiency when simultaneously learning or predicting the plurality of health indicators, and may make it possible to optimize the model performance while saving computing resources.

Next, the output generation module 112 may receive the feature vector generated by the feature extraction module 111 as an input, and may generate a prediction result for each health indicator. The output generation module 112 may include a plurality of head sub-models, and may perform a prediction optimized to suit the characteristics of each health indicator. Although the feature vector generated by the feature extraction module 111 includes a common feature, since each health indicator has a different clinical meaning and pattern, a sub-model specialized for each indicator may be required to independently analyze and predict it.

The output generation module 112 may be configured such that the plurality of head sub-models operate in parallel to simultaneously predict all the health indicators. This maximizes the learning and prediction efficiency, and can derive an optimal prediction result reflecting the specificity of each indicator while utilizing a common feature representation. Through this, by performing prediction for various health indicators in one model structure, accuracy and clinical reliability can be simultaneously secured. For example, the output generation module 112 may generate a plurality of health indicators (for example, a cardiovascular health indicator, a kidney health indicator, blood pressure, and the like) predicted in relation to diseases having high mutual relevance with respect to the current health condition of the subject. In addition, the output generation module 112 may selectively extract a health indicator included in a specific disease among the plurality of health indicators, and may output the extracted health indicator. For example, the health indicator to be extracted may be predetermined, or may be received from the outside.

In addition, the second model 120 may perform a role of evaluating the health risk of the individual by utilizing the health indicators, which are the output values of the first model 110. The second model 120 may be designed on a statistical basis, and may analyze the correlation between the health indicators and the health risk by utilizing various statistical techniques such as the Cox proportional hazards model (hereinafter expressed as the Cox model) and logistic regression. The second model 120 may receive the health indicators output from the first model 110 (for example, a cardiovascular health indicator, a kidney health indicator, blood pressure, and the like) as an input value, analyze the relationship therebetween, and calculate the health risk degree.

For example, the Cox model is a statistical technique suitable for survival analysis, and may be used to evaluate the risk degree of occurrence of a major cardiovascular event (for example, myocardial infarction, stroke) during a specific period. The Cox model mathematically analyzes the proportional hazard relationship between the health indicator and the occurrence of the event, and may dynamically calculate the risk degree by including a time variable. That is, the output value of the second model 120 is a result of comprehensively evaluating the health risk of the individual, and may be provided in a form such as, for example, "the risk degree of occurrence of a major cardiovascular disease event within 5 years" or "the probability that the cardiovascular disease risk is 10% or more."

The first model 110 or the second model 120 may receive, as an additional input, not only the health indicator generated from the medical image data but also other data of the individual, and may combine them to perform a more precise health risk analysis. The data of the individual that may be utilized as the additional input includes various information related to the health condition in addition to the medical image data. For example, the data of the individual that may be utilized as the additional input, that is, the additional data, may include clinical data such as age, gender, body mass index (BMI), smoking status, family history, blood pressure, and blood glucose level. Such additional data, together with the health indicator derived from the medical image, may contribute to more comprehensively and accurately analyzing the health risk of the individual.

In the first model 110, the additional data may be utilized as an auxiliary input for health indicator prediction. For example, when the processor inputs blood pressure information together with the fundus image to the first model 110 to predict the CAC score, the blood pressure information acts as an additional feature considering the correlation with the vessel condition, thereby increasing the accuracy of the prediction.

In the second model 120, the additional data may be combined and utilized in the health risk analysis process. For example, the processor may use the health indicators such as the CAC score and the eGFR generated by the first model 110 as main input values of the Cox model of the second model 120, while additionally inputting data such as age, gender, and family history to the second model 120 to integrally analyze them. Through such combination, the processor can more deeply understand the overall health condition of the individual by using the second model 120, and can more precisely predict a result such as, for example, the risk of occurrence of a cardiovascular event within 5 years. In addition, the additional data input to the second model 120 may be predicted as one of the plurality of health indicators by the first model 110, or may be received from the outside.

When described using a specific embodiment, the processor of the diagnostic apparatus may input a fundus image of the subject to the feature extraction module 111 of the first model 110 of the health risk prediction model 100. The processor may extract a feature vector of the input fundus image by using the feature extraction module 111, and may input the extracted feature vector to the output generation module 112. Then the processor may generate a plurality of health indicators in the output generation module 112. The processor may select and extract, as a cardiovascular health indicator among the plurality of health indicators, a probability value that the CAC score is greater than 0. In addition, the processor may input, to the second model 120, the probability value that the CAC score extracted by the output generation module 112 is greater than 0, together with age and gender. At this time, the age and gender may be selected and output as a health indicator in the output generation module 112, or may be received from the outside. The processor may generate the risk degree of occurrence of a major cardiovascular disease event within 10 years by using the second model 120, and may output it.

In addition, when the processor selects and extracts, as a kidney health indicator among the plurality of health indicators, a probability value that the eGFR is less than 60, the processor may input, to the second model 120, the probability value that the eGFR extracted by the output generation module 112 is less than 60, together with age and gender. Then the processor may generate the risk degree of occurrence of a major kidney disease event within 10 years by using the second model 120, and may output it.

### Labeling of a plurality of health indicators based on clinical thresholds

The plurality of health indicators may be defined as labels classified based on clinically important reference values (thresholds, clinical thresholds) in order to evaluate the health condition of the individual from various angles. Each health indicator is divided according to an intrinsic threshold directly related to clinical judgment, and such a labeling scheme may be designed so that the prediction result is clinically interpretable. Here, the threshold may be based on medical evidence and research data, or may be based on a guideline announced by an international or regional medical institution (for example, the American Heart Association (AHA), the World Health Organization (WHO), the Korean Academy of Medical Sciences, and the like) on the basis of the latest research results and expert consensus. Alternatively, it may be a value determined based on the results of a large-scale clinical study.

In an embodiment, the training apparatus may train the health risk prediction model 100 by using the medical image data labeled according to the clinical thresholds.

The scheme of labeling each health indicator according to its intrinsic clinical threshold may act as an important tool capable of subdividing and evaluating the risk level for each disease. For example, the coronary artery calcium score (CAC score) is a main indicator for evaluating the risk of coronary artery disease, and may indicate the risk degree of the disease in stages by being classified into sections such as CAC score = 0, 0 < CAC score ≤ 100, 100 < CAC score ≤ 300, and CAC score > 300. Similarly, the kidney function indicator (eGFR) is divided on the basis of thresholds such as eGFR ≥ 90, 60 ≤ eGFR < 90, 30 ≤ eGFR < 60, and eGFR < 30, and this may be useful for evaluating the severity of kidney disease and subdividing the patient state. Such a plurality of labels may contribute to allowing medical staff to more clearly understand the disease state of the patient and to establish an appropriate treatment plan.

In addition, each health indicator may be composed of a plurality of binary labels according to its intrinsic threshold. The binary label determines whether the patient is at or above or at or below a specific risk level on the basis of the threshold, and may provide an advantage of intuitively analyzing the possibility of occurrence of an event. For example, the CAC score may be configured as a binary label of whether the CAC score is greater than 0 or 0, a binary label of whether the CAC score is greater than 100 or 100 or less, and/or a binary label of whether the CAC score is greater than 300 or 300 or less, so that it can be simply confirmed whether the patient falls into a high-risk group within a specific risk section. Similarly, the eGFR may be labeled as a binary label of whether the eGFR is less than 90 or greater than 90, a binary label of whether the eGFR is less than 60 or greater than 60, a binary label of whether the eGFR is less than 30 or greater than 30, and a binary label of whether the eGFR is less than 15 or greater than 15, so that the deterioration state of the kidney function can be divided in stages. As an example, the following Table 1 shows an example of the clinical thresholds and the label ranges of the labels used for training for cardiovascular disease and kidney disease.

In an embodiment, the training apparatus may train the health risk prediction model 100 by using the medical image data labeled in a binary labeling scheme.

**[Table 1]**

| **Disease** | **Label type** | **Clinical threshold** | **Label range (binary label)** | **Related clinical content** |
|---|---|---|---|---|
| CVD (cardiovascular disease) | CAC score | 1) Low: CAC score =0 | 1) CAC score > 0 or CAC score = 0 | The CAC (coronary artery calcium) score is classified into 5 risk groups according to clinical thresholds. |
| | | 2) Mild: 0 < CAC score ≤ 100 | 2) CAC score > 100 or CAC score ≤ 100 | |
| | | 3) Moderate: 100 < CAC score ≤ 300 | | |
| | | | 3) CAC score > 300 or CAC score ≤ 300 | |
| | | 4) Severe: 300 < CAC score ≤ 1000 | | |
| | | 5) Extreme: CAC score > 1000 | | |
| CKD (kidney disease) | eGFR | 1) Normal: eGFR 90 or more | 1) eGFR less than 90 or eGFR greater than 90 | The eGFR (estimated glomerular filtration rate) is classified to define the stages of chronic kidney disease (CKD) according to the guidelines of the National Kidney Foundation (NKF). |
| | | 2) Mildly decreased: eGFR 60 to 89 | | |
| | | | 2) eGFR less than 60 or eGFR greater than 60 | |
| | | 3) Moderately decreased: eGFR 30 to 59 | | |
| | | | 3) eGFR less than 30 or eGFR greater than 30 | |
| | | 4) Severely decreased: eGFR 15 to 29 | | |
| | | | 4) eGFR less than 15 or eGFR greater than 15 | |
| | | 5) Kidney failure: eGFR less than 15 | | |

Such a binary labeling scheme may have the following usefulness.

First, the interpretation of the prediction result is simple and clear, so that medical staff can quickly evaluate a specific risk level. For example, a high-risk group (Severe) patient such as CAC score > 300 can be quickly identified, and a measure accordingly can be immediately planned.

Second, the binary label is efficient for the health risk prediction model to learn the possibility of occurrence of an event. A scheme of determining whether it is at or above or at or below a single reference value, such as the binary labeling scheme, is simpler to learn than a multi-section label, and may contribute to increasing the generalization performance of the model.

Finally, the binary label is suitable for comparing clinical data of patients or analyzing tendencies, and may also be advantageous for analyzing large-scale population data to identify the characteristics of a specific risk group.

The scheme of configuring each health indicator as a plurality of labels or binary labels according to its intrinsic clinical threshold may exert a more powerful effect when combined with multitask learning, which will be described later.

### Multitask Learning

The first model 110 is a neural network-based model designed to receive a medical image as an input and predict a plurality of health indicators, and the prediction performance and efficiency may be maximized through various learning techniques by the first model 110. In particular, the training apparatus may train the first model 110 by using a multitask learning scheme for simultaneously learning a plurality of health indicators. In the multitask learning scheme, the common feature vector extracted from the medical image data may be shared by the plurality of health indicators, and thereby the learning efficiency of the first model 110 may be increased.

Specifically, the feature extraction module 111 learns the common pattern and information in the medical image, and the feature vector generated thereby may be delivered to the output generation module 112 specialized for each health indicator. Each head sub-model included in the output generation module 112 performs a prediction task specialized for each health indicator, and may further optimize the prediction performance by utilizing the correlation between the plurality of health indicators. Through such multitask learning, the first model 110 may be capable of efficient and consistent learning compared to single-task learning.

The data labeled on the basis of the intrinsic clinical threshold clearly reflects the clinical meaning of each health indicator, and enables a plurality of health indicators to be simultaneously learned and predicted through multitask learning. The multitask learning, by having the common feature vector extracted from the medical image data shared by the several health indicators, can integrally reflect the correlation between the indicators in the model. For example, the coronary artery calcium score (CAC score) and the kidney function indicator (eGFR) are respectively used to predict coronary artery disease and kidney disease, but the two indicators may share common physiological characteristics such as vessel condition and tissue damage. The multitask learning, by learning such common features, can increase not only the prediction accuracy of the individual health indicators but also the reliability of the overall health risk assessment.

In particular, the labeling based on the intrinsic clinical threshold can maximize the performance of the multitask learning. An indicator such as the CAC score is classified into sections such as CAC score = 0, 0 < CAC score ≤ 100, 100 < CAC score ≤ 300, and CAC score > 300 to subdivide the risk degree of each stage, and the eGFR is divided into eGFR ≥ 90, 60 ≤ eGFR < 90, 30 ≤ eGFR < 60, and eGFR < 30 to reflect the severity of kidney disease in stages. Such labeling may help the first model 110 to learn clinically important reference values, and thereby the health risk prediction model 100 may provide a prediction result usable in the medical field. At the same time, when each indicator is composed of a binary label such as a binary label of whether the CAC score is greater than 0 or 0, a binary label of whether the CAC score is greater than 100 or 100 or less, and/or a binary label of whether the CAC score is greater than 300, or a binary label of whether the eGFR is less than 90 or greater than 90, a binary label of whether the eGFR is less than 60 or greater than 60, a binary label of whether the eGFR is less than 30 or greater than 30, and/or a binary label of whether the eGFR is less than 15 or greater than 15, whether it is at or above a specific risk level can be simply determined, so that the efficiency and interpretability of the model learning may be greatly improved. Hereinafter, the multitask model will be described in more detail.

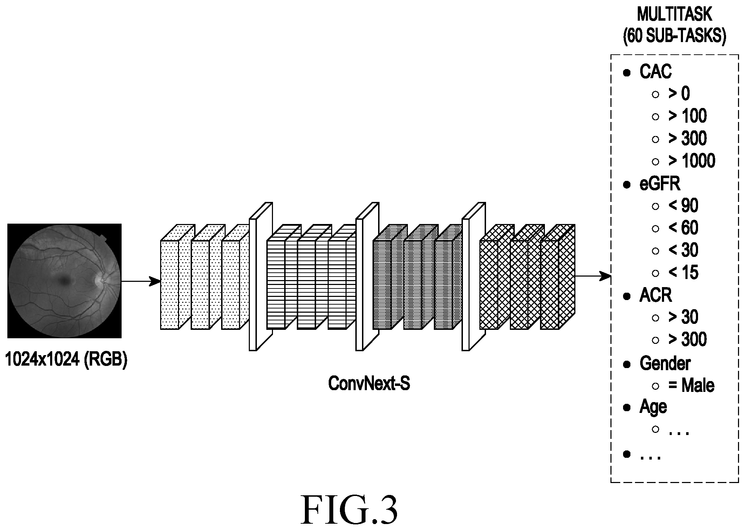

FIG. 3 is a diagram for explaining multitask learning according to an embodiment.

Referring to FIG. 3, in the embodiment of FIG. 3, an embodiment in which a fundus image among the medical image data is input to the first model will be described.

Specifically, a CNN (convolutional neural network) model (for example, a ConvNext-S model) may be applied to the first model of the health risk prediction model, and the training apparatus may simultaneously learn 21 health indicators together with the fundus image with respect to the first model. The health indicators are binary-classified on the basis of clinical thresholds, and for example, learning for a total of 60 binary classification sub-tasks may be simultaneously performed in a multitask manner.

The following Table 2 relates to the sub-tasks for each health indicator (task). The 21 health indicators presented in the following Table 2 are divided into binary labels according to the clinical meaning of each indicator, and through this, the model can learn the correlation between the indicators and at the same time improve the prediction performance of the individual indicators.

**[Table 2]**

| **Health indicator (task)** | **Sub-task** |
|---|---|
| CAC score (CAC_score) | CAC_score > 0, CAC score > 100, CAC score > 300, CAC_score > 1000 |
| eGFR | eGFR < 90, eGFR < 60, eGFR < 30, eGFR < 15 |
| Age | Age > 40, Age > 50, Age > 60, Age > 70 |
| Gender | Gender == Male |
| BMI | BMI >= 18.5, BMI >= 25, BMI > 30 |
| Systolic blood pressure (SBP) | SBP > 120, SBP > 130, SBP > 140 |
| Diastolic blood pressure (DBP) | DBP > 80, DBP > 90 |
| Blood urea nitrogen (BUN) | BUN > 6, BUN > 20 |
| Cholesterol | Cholesterol > 200, Cholesterol > 240 |
| Creatinine | Creatinine > 0.5, Creatinine > 1.2, Creatinine > 1.4 |
| Glucose | Glucose > 70, Glucose > 100, Glucose > 126 |
| HDL cholesterol (HDL) | HDL < 60, HDL < 40 |
| LDL cholesterol (LDL) | LDL > 100, LDL > 130, LDL > 160, LDL > 190 |
| Triglyceride | Triglyceride > 150, Triglyceride > 200, Triglyceride > 250 |
| Albumin-creatinine ratio (Albumin_Creatinine_Ratio, ACR) | Albumin_creatinine_ratio > 30, Albumin_creatinine_ratio > 300 |
| Glycated hemoglobin (Hbalc) | Hbalc > 5.7, Hbalc > 6.5, Hbalc > 7.5, Hbalc > 8.5 |
| Albumin | Albumin < 3.5 |
| Smoking | (Smoking == "Previous" or Smoking == "Current"), Smoking == "Current" |
| Body fat percentage (Body_fat) | Body_fat > 6, Body_fat > 14, Body > 18, Body > 21, Body_fat > 25, Body_fat > 32 |
| Height | Height > 167 |
| Weight | Weight > 55, Weight > 70, Weight > 85, Weight > 100 |

**As** shown in Table 2 above, among the respective health indicators (the respective tasks), the CAC score may include a sub-task of a binary label of whether the CAC score is greater than 0 or 0, a sub-task of a binary label of whether the CAC score is greater than 100 or 100 or less, a sub-task of a binary label of whether the CAC score is greater than 300 or 300 or less, and a sub-task of a binary label of whether the CAC score is greater than 1000 or 1000 or less. In addition, the eGFR may include a sub-task of a binary label of whether the eGFR is less than 90 or greater than 90, a sub-task of a binary label of whether the eGFR is less than 60 or greater than 60, a sub-task of a binary label of whether the eGFR is less than 30 or greater than 30, and/or a sub-task of a binary label of whether the eGFR is less than 15 or greater than 15. The training apparatus may train the first model by independently using such approximately 60 sub-tasks. For example, the training apparatus may perform learning in a direction in which the loss of the predicted value for a first sub-task of the first model (for example, the sub-task of the binary label of whether the CAC score is greater than 0 or 0) is lowered, and may sequentially perform learning in a direction in which the loss of the predicted value for a second sub-task (for example, the sub-task of the binary label of whether the CAC score is greater than 100 or 100 or less) is lowered. In addition, sequentially, for each of the 60 sub-tasks, learning may be performed in a direction in which the loss of the predicted value for each sub-task is lowered. By such a multitask learning scheme, information of all the sub-tasks may be reflected in the first model.

Such a combination of the intrinsic labeling scheme and the multitask learning, by learning the mutually complementary information between the respective health indicators, provides higher prediction accuracy even in new patient data, improves the generalization performance, and can improve the compatibility between medical images.

The following Table 3 shows the cardiovascular disease risk degree prediction and kidney disease risk degree prediction performance of the health risk prediction model including the first model trained in the multitask learning scheme. As a result of the analysis, compared to a cardiovascular risk prediction model that learned the fundus image and the single indicator CAC > 0 label, the health risk prediction model including the first model trained with the 21 health indicators (60 sub-tasks) in the multitask learning scheme had a prediction performance improved by about 9%. In addition, compared to a kidney disease risk prediction model that learned the fundus image and the single indicator eGFR < 60 label, the health risk prediction model including the first model trained in the multitask learning scheme had a prediction performance improved by about 6%.

**[Table 3]**

| **Cardiovascular disease risk assessment** | **Kidney disease risk assessment** |
|---|---|
| When using only CAC > 0: Prediction C-index: **65.27%** | When using only eGFR < 60: Prediction C-index: **71.62%** |
| When utilizing all labels: Prediction C-index: **71.27%** | When utilizing all labels: Prediction C-index: **75.81%** |

### Self-training Technique

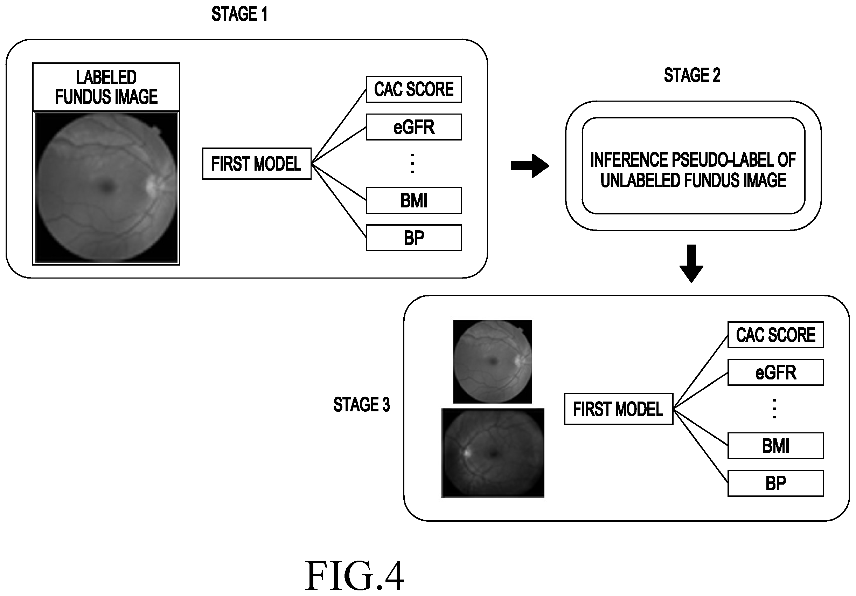

FIG. 4 is a diagram for explaining self-training according to an embodiment.

Referring to FIG. 4, the case where all of the plurality of health indicators necessary for learning are labeled in the medical image data for a patient may be limited. For example, as can be seen in Table 4 below, in relation to cardiovascular disease prediction, the data in which the CAC score is labeled together with the fundus image is only 63,909, but the data in which one or more of the 21 health indicators is labeled together with the fundus image reaches 639,665. Similarly, in relation to kidney disease prediction, the data in which the eGFR is labeled together with the fundus image is only 184,771, but the data in which one or more of the health indicators is labeled may be confirmed as 639,664.

**[Table 4]**

| **Disease** | **Number of medical data having only the CAC label or the eGFR label** | **Number of medical data in which one or more of the 21 health indicators is labeled** | **Degree of increase** |
|---|---|---|---|
| CVD (CAC label) | 64,909 | 649,665 | 10.01-fold increase |
| CKD (eGFR label) | 184,771 | 649,665 | 3.52-fold increase |

In order to solve the problem that cases in which all of the plurality of health indicators are labeled are limited, the training apparatus may utilize a self-training technique to perform additional learning on data lacking labels. In an embodiment, the self-training technique may proceed as follows. First, when ground truth labels for some health indicators are insufficient in the medical image data, the predicted value of the corresponding health indicator may be generated by using the already-trained first model. Thereafter, highly reliable data may be selected from among the generated predicted values, and may be regarded as a temporary ground truth label of the corresponding health indicator so that the first model is additionally trained. Here, the temporary ground truth label may be expressed as an inference pseudo-label.

For example, when the medical image data is not labeled with respect to the eGFR among the 21 health indicators above, the training apparatus may input the corresponding medical image data to the first model to generate the predicted value of the eGFR. For example, the training apparatus may generate, for the corresponding medical image data, a predicted value for the binary label of whether the eGFR is less than 90 or greater than 90, a predicted value for the binary label of whether the eGFR is less than 60 or greater than 60, a predicted value for the binary label of whether the eGFR is less than 30 or greater than 30, and a predicted value for the binary label of whether the eGFR is less than 15 or greater than 15. In addition, the training apparatus may select the predicted value having the highest reliability among the generated predicted values. For example, when the predicted value that the eGFR is greater than 90 has the highest reliability, the training apparatus may regard the eGFR of the corresponding medical image data as being greater than 90 and additionally train the first model.

Such a self-training technique may be very useful in consideration of the characteristics of medical data for which the labeling cost is high. According to the self-training technique, not only can the data lacking labels be effectively utilized, but it can also contribute to improving the generalization performance of the first model. As a result, the self-training technique plays an important role in increasing the learning efficiency and performance of the first model that predicts a plurality of health indicators by maximally utilizing the medical image data.

### Medical images captured under different conditions

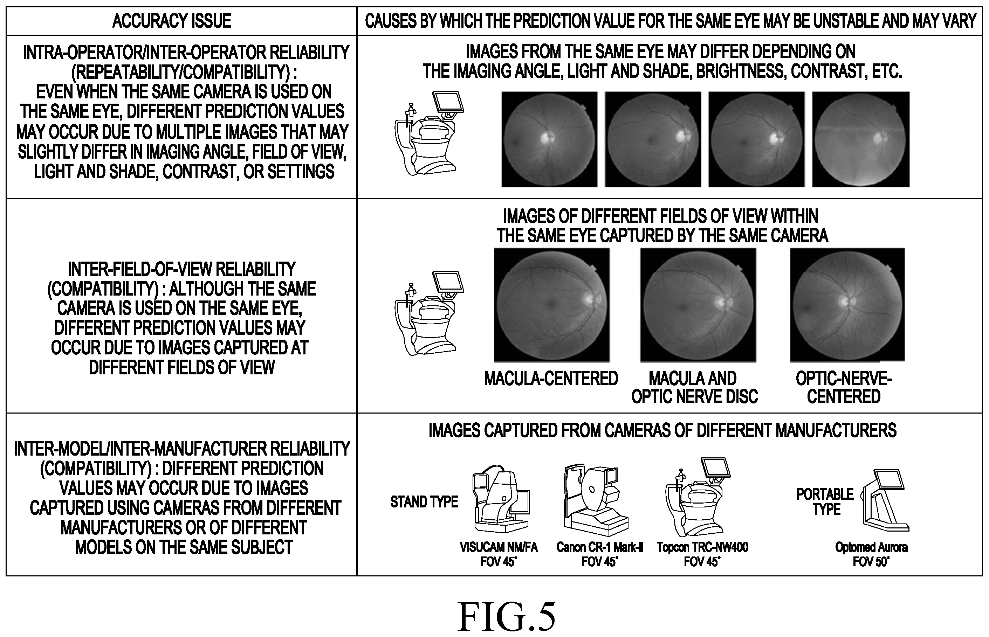

FIG. 5 is a diagram for explaining prediction performance for medical images captured under different conditions according to an embodiment.

Referring to FIG. 5, medical images may be captured under different conditions even for the same subject. Here, the different conditions may include the type of the camera that captures the medical image, the field of view of the camera, whether a mydriatic agent has been applied to the subject, whether the imaging target is the left eye or the right eye, the operator of the camera and/or the imaging environment (for example, whether the surroundings of the camera are dark or bright), and the like. In addition, the type of the camera may include the camera manufacturer, the camera model, and/or the type of the camera (for example, a stand type or a portable type).

For example, as in FIG. 5, even when the same eye of the subject is captured with the same camera, if the imaging angle, the field of view, the light and shade, the contrast, or the settings are different, different fundus images may be generated, and as a result, different predicted values may occur. As an example, capturing with different fields of view may mean whether the camera captures centered on the macula, centered on the macula and the optic nerve disc, or centered on the optic nerve.

In addition, even when the same eye of the subject is captured with different cameras (for example, cameras of different manufacturers, or cameras of different models even from the same manufacturer), different fundus images may be generated, and as a result, different predicted values may occur.

Such different conditions may cause problems of compatibility and reproducibility of the prediction results. The compatibility problem refers to a problem of not producing consistent results in different devices, environments, and conditions. This mainly occurs due to differences between equipment or environments, and is an important consideration in medical image analysis or an AI-based diagnostic system. The conditions that can be classified as compatibility problems are as follows.
1) Type of camera: manufacturer difference (e.g., camera of company A and camera of company B), model difference (e.g., difference in a specific resolution or sensor performance).
2) Difference in camera type: stand-type vs. portable camera.
3) Difference in field of view of the camera: difference in camera design characteristics such as a wide field of view and a narrow field of view.
4) Difference in imaging target: physical structural or environmental difference between the left eye and the right eye.
5) Difference in imaging environment: difference in external lighting conditions such as a dark environment and a bright environment.

In addition, the reproducibility problem refers to a problem in which the results are not consistent even in the same device or condition. This mainly corresponds to a case where repeated measurements or work results in the same environment show variability. The conditions that can be classified as reproducibility problems are as follows.
1) Difference in operator: result variation due to the difference in the operator's proficiency or method even with the same camera.
2) Whether a mydriatic agent is applied: difference in results depending on whether a mydriatic agent is used even for the same subject.
3) Difference in imaging environment: even when the same camera is used, the results may differ depending on whether the surrounding environment is dark or bright.

However, since the terms compatibility and reproducibility are often difficult to strictly distinguish in practice, there are cases where they are used interchangeably. The term "compatibility" described in the present specification is defined as a concept including reproducibility.

### Statistics-based stabilization method

In order to solve the problem that different predicted values occur in the medical images captured under different conditions as described above, the present invention proposes a statistics-based stabilization method. A prediction score according to the statistics-based stabilization method may also be expressed as a Stabilized Deep Learning Risk Score (DLRS). The statistics-based stabilization method is a method of combining a Cox model with a deep learning-based prediction score to reinforce the stability and clinical reliability of the prediction. Here, the deep learning-based prediction score may mean a probability value output from the above-described first model. An existing deep learning-based prediction score was calculated based only on medical image data, but a prediction score according to the statistics-based stabilization method integrates clinical factors such as the patient's age and gender together with the deep learning-based prediction score, so that the prediction result can be adjusted more precisely.

In addition, compared with a Cox model utilizing only the deep learning prediction score, this method can provide a more sophisticated risk score reflecting the clinical meaning. In particular, the prediction score according to the statistics-based stabilization method calculated through the equation of the Cox model can predict a long-term health risk such as the 10-year cardiovascular disease (CVD) occurrence risk with high reliability.

In an embodiment, the Cox model integrates the patient's clinical factors together with the existing deep learning prediction score (S), so that the 10-year cardiovascular disease (CVD) occurrence risk can be calculated by the following Equation 1. [Equation 1] $CVD risk \left(10\left|S\right.\right) = 1 - exp \left\{-{A}_{0}\left(10\right)exp\left\{β′\times S+β{′}_{Age}\times Age+β{′}_{Gender}\times I\left(Male\right)\right\}\right\}$

Here, CVD risk(10|S) represents a probability value for the occurrence probability of a cardiovascular-disease-related event of the subject within 10 years, A₀(10) represents a baseline cumulative function on a 10-year basis, S represents a probability value output from the first model with respect to the medical image data when the medical image data of the subject is input to the first model, Age represents the age of the subject, and I(Male) represents the gender of the subject. For example, I(Male) may be an indicator function that reflects a value of 1 when the subject is male and reflects a value of 0 when the subject is female.

In addition, β' represents a regression coefficient for the output value output from the first model, β'_{Age} represents a regression coefficient for the age, and β'_{Gender} represents a regression coefficient for the gender. In addition, the values of the regression coefficients may be adjusted through the fitting process of the second model in the learning model.

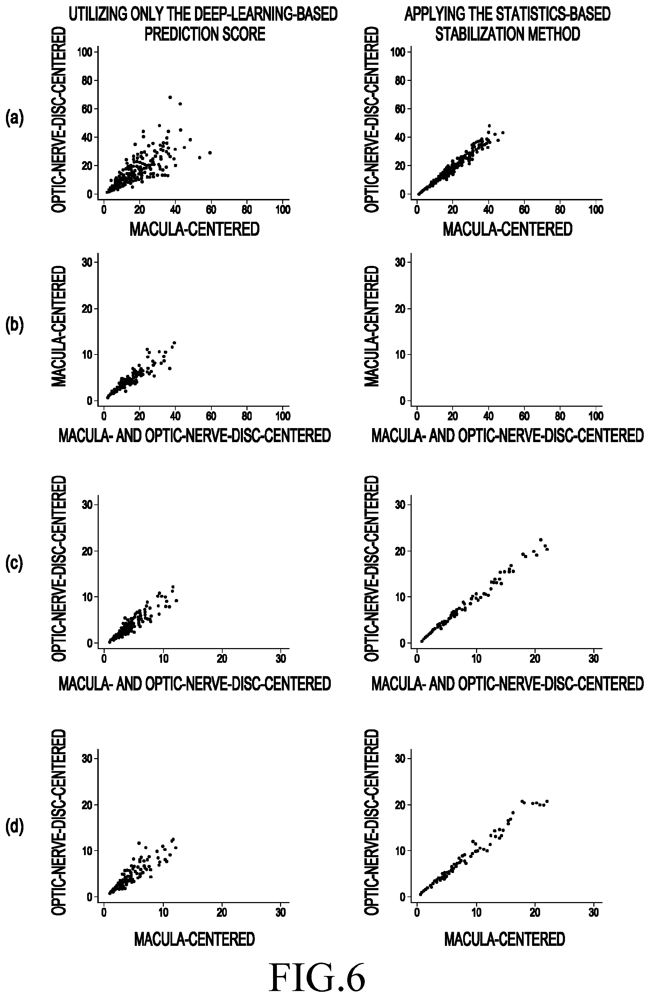

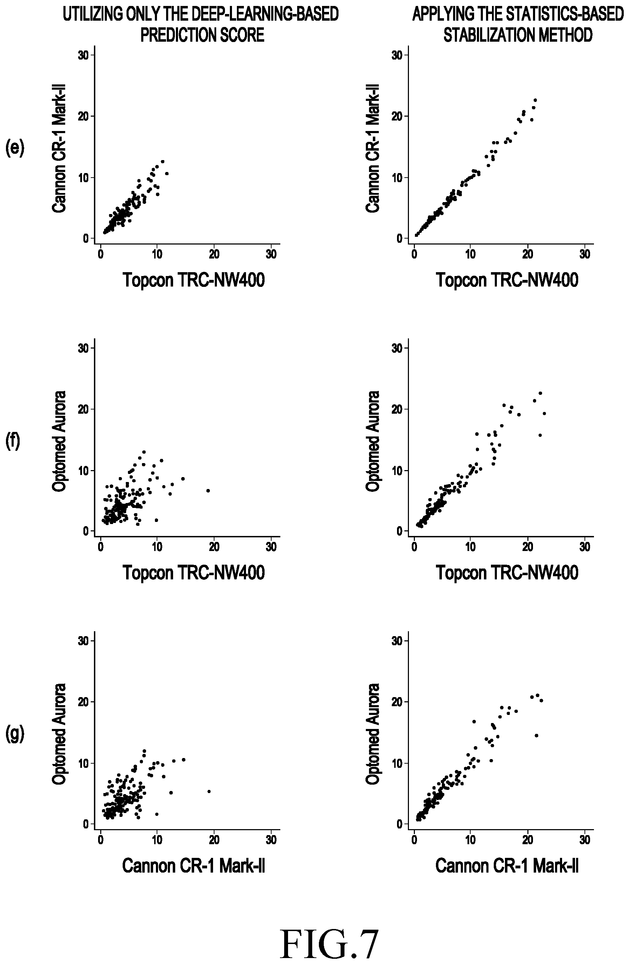

The prediction score according to the statistics-based stabilization method was verified using various external datasets (UK Biobank, CMERC-HI, SILOAM), and showed a performance improvement of 6 to 10% or more compared with the existing model in indicators such as AUC and C-Index. Through this, the present method proves that high generalization performance and stability can be maintained even in various clinical environments based on medical image data. This will be described in detail using FIG. 6 and FIG. 7.

FIGS. 6 and 7 are diagrams for explaining prediction performance according to a statistics-based stabilization method according to an embodiment.

Referring to FIGS. 6 and 7, in (a) to (g), the graphs on the left represent the graphs when only the deep learning-based prediction score is utilized in the COX model, and the graphs on the right represent the graphs when the statistics-based stabilization method is applied. Here, that only the deep learning-based prediction score is utilized in the COX model represents a case where, in the above-described Equation 1, only S, that is, the probability value output from the first model, is reflected, and the age and gender are not reflected. In addition, that the statistics-based stabilization method is applied may represent a case where, in the above-described Equation 1, not only the probability value output from the first model, but also both the age and gender are reflected.

In addition, the following Table 5 summarizes the demographic population of the medical image data utilized in FIG. 6 and FIG. 7. In the following Table 5, CMERC-HI is an abbreviation of Cardiovascular and Metabolic Disease Etiology Research Center-High Risk, and SILOAM may represent an abbreviation of Simple Low-cost One-stop AI Multiorgan screening. In addition, ASCVD may represent an abbreviation of Atherosclerotic Cardiovascular Disease.

**[Table 5]**

| **Variable** | **UK Biobank** | **CMERC-HI** | **SILOAM** |
|---|---|---|---|
| Number of subjects, n | 45,473 | 957 | 160 |
| Camera | Topcon 3D OCT-1000 Mark II | Carl Zeiss VISUCAM NM/FA | Topcon TRC-NW400, Canon CR-1 Mark-II, Optomed Aurora |
| Number of medical images, n | | | |
| Macula-centered, n | 90,946 | 1,914 | 318 (Topcon), 320 (Canon), 314 (Optomed) |
| Macula- and | Not available | Not available | 320 |
| optic-nerve-disc field of view, n | | | |
| Optic-nerve-disc-centered, n | Not available | 1,914 | 314 |
| Age, mean (standard deviation) | 56.1 (8.2) | 58.3 (12.1) | 56.7 (8.1) |
| Number of females, n (%) | 24,278 (53.4) | 423 (44.2) | 96 (60%) |
| Number of ASCVD, | 2,522 (5.5) | Not available | Not available |

Specifically, FIG. 6 represents a scatter plot of the prediction scores when only the deep learning-based prediction score is utilized in the COX model and the prediction scores when the statistics-based stabilization method is applied, with respect to medical images captured at different fields of view. In addition, the following Table 6 summarizes the contents of the graphs of FIG. 6 in a table.

**[Table 6]**

| **Variable** | **Comparison image** | **Correlation when utilizing only deep-learning-based prediction score** | **Correlation when applying statistics-based stabilization method** |
|---|---|---|---|
| CMERC-HI | | | |
| Optic-nerve-disc-centered | Macula-centered | 0.866 | 0.988 |

| SILOAM | | | |
|---|---|---|---|
| Macula- and optic-nerve-disc field of view | Macula-centered | 0.939 | 0.994 |
| Macula-centered | Optic-nerve-disc-centered | 0.890 | 0.990 |
| Optic-nerve-disc-centered | Macula- and optic-nerve-disc field of view | 0.931 | 0.995 |

(a) relates to the dataset of CMERC-HI, and (b) to (d) relate to the dataset of SILOAM. For example, the left graph of (a) represents the correlation between the prediction score when only the deep learning-based prediction score is utilized in the COX model with respect to the optic-nerve-disc-centered medical image and the deep learning-based prediction score with respect to the macula-centered medical image, and the right graph represents the correlation between the prediction score when the statistics-based stabilization method is applied with respect to the optic-nerve-disc-centered medical image and the prediction score when the statistics-based stabilization method is applied with respect to the macula-centered medical image.

In addition, the following Table 7 represents the ICC (Intraclass Correlation Coefficient) of the prediction score when only the deep learning-based prediction score is utilized in the COX model and the ICC of the prediction score when the statistics-based stabilization method is applied.

**[Table 7]**

| **Dataset / Medical image** | **Prediction score** | **ICC (95% CI)** |
|---|---|---|
| CMERC-HI | | |
| Macula-centered vs. optic-nerve-disc-centered | Utilizing only deep-learning-based prediction score | 0.861 (0.838 - 0.880) |
| | Applying statistics-based stabilization method | 0.988 (0.986 - 0.989) |

| SILOAM | | |
|---|---|---|
| All images captured at 3 fields of view | Utilizing only deep-learning-based prediction score | 0.917 (0.893 - 0.937) |
| | Applying statistics-based stabilization method | 0.993 (0.991 - 0.995) |
| Macula- and optic-nerve-disc field of view vs. macula-centered | Utilizing only deep-learning-based prediction score | 0.939 (0.917 - 0.955) |
| | Applying statistics-based stabilization method | 0.994 (0.992 - 0.996) |
| Macula- and optic- | Utilizing only deep- | 0.928 (0.900 - |
| nerve-disc field of view vs. optic-nerve-disc-centered | learning-based prediction score | 0.948) |
| | Applying statistics-based stabilization method | 0.994 (0.992 - 0.996) |
| Macula-centered vs. optic-nerve-disc-centered | Utilizing only deep-learning-based prediction score | 0.884 (0.842 - 0.915) |
| | Applying statistics-based stabilization method | 0.990 (0.986 - 0.993) |

As shown in (a) to (d), Table 6, and Table 7, the correlation of the prediction score when only the deep learning-based prediction score is utilized in the COX model is shown to be higher than the correlation of the prediction score when the statistics-based stabilization method is applied. In addition, compared with the ICC (Intraclass Correlation Coefficient) of the prediction score when only the deep learning-based prediction score is utilized in the COX model, the ICC of the prediction score when the statistics-based stabilization method is applied is shown to be higher.

As such, the performance improvement of the statistics-based stabilization method was consistently observed in various datasets.

In addition, FIG. 7 represents a scatter plot of the prediction scores when only the deep learning-based prediction score is utilized in the COX model and the prediction scores when the statistics-based stabilization method is applied, with respect to medical images captured with different camera models. In addition, the following Table 8 summarizes the contents of the graphs of FIG. 7 in a table.

**[Table 8]**

| **Variable** | **Comparison image** | **Correlation when utilizing only deep-learning-based prediction score** | **Correlation when applying statistics-based stabilization method** |
|---|---|---|---|
| SILOAM | | | |
| Topcon TRC-NW400 | Canon CR-1 Mark-II | 0.942 | 0.995 |
| Canon CR-1 Mark-II | Optomed Aurora | 0.610 | 0.972 |
| Optomed Aurora | Topcon TRC-NW400 | 0.579 | 0.972 |

(e) relates to the dataset captured with Canon's CR-1 Mark-II (stand type) and the dataset captured with Topcon's TRC-NW400 (stand type). In addition, (f) relates to the dataset captured with Optomed's Aurora (portable type) and the dataset captured with Topcon's TRC-NW400, and (g) relates to the dataset captured with Optomed's Aurora (portable type) and the dataset captured with Canon's CR-1 Mark-II (stand type). For example, the left graph of (e) represents the correlation between the prediction score when only the deep learning-based prediction score is utilized in the COX model with respect to the medical image captured with Canon's CR-1 Mark-II (stand type) and the deep learning-based prediction score with respect to the medical image captured with Topcon's TRC-NW400, and the right graph represents the correlation between the prediction score when the statistics-based stabilization method is applied with respect to the medical image captured with Canon's CR-1 Mark-II (stand type) and the prediction score when the statistics-based stabilization method is applied with respect to the medical image captured with Topcon's TRC-NW400.

In addition, the following Table 9 represents the ICC of the prediction score when only the deep learning-based prediction score is utilized in the COX model and the ICC of the prediction score when the statistics-based stabilization method is applied.

**[Table 9]**

| **Dataset / Medical image** | **Prediction score** | **ICC (95% CI)** |
|---|---|---|
| SILOAM | | |
| All images captured with 3 cameras | Utilizing only deep-learning-based prediction score | 0.534 (0.243 - 0.710) |
| | Applying statistics-based stabilization method | 0.965 (0.926 - 0.981) |
| Topcon vs. Canon | Utilizing only deep-learning-based prediction score | 0.933 (0.892 - 0.957) |
| | Applying statistics-based stabilization method | 0.965 (0.919 - 0.985) |
| Topcon vs. Optomed | Utilizing only deep-learning-based prediction score | 0.436 (0.061 - 0.659) |
| | Applying statistics-based stabilization method | 0.956 (0.877 - 0.978) |
| Canon vs. Optomed | Utilizing only deep-learning-based prediction score | 0.420 (0.015 - 0.672) |
| | Applying statistics-based stabilization method | 0.949 (0.814 - 0.978) |

As shown in (e) to (g), Table 8, and Table 9, as a result of analyzing the correlation coefficient (Corr) and ICC between the medical image data captured with cameras of various manufacturers such as Optomed, Canon, and Topcon and cameras of various types (stand type and portable type), the prediction score when the statistics-based stabilization method is applied recorded a high correlation (0.97 or more) and a high ICC (0.94) regardless of the camera manufacturer and the camera type. This indicates that, when the statistics-based stabilization method is applied, the difference between cameras can be effectively corrected compared with the existing method. In particular, it maintains consistent performance even in datasets obtained from various cameras, indicating that the camera compatibility can be greatly improved in an actual clinical environment.

In summary, in medical image analysis, differences in the camera manufacturer, the imaging environment, and the resolution are major factors that affect the consistency and reliability of the data. The statistics-based stabilization method of the present invention can correct the difference between manufacturers by utilizing an integrated risk score based on the Cox model in order to effectively solve such problems. In addition, the performance difference between images captured with portable cameras such as Optomed's camera and images captured with stand-type cameras of Canon and Topcon showed an ICC difference of up to 0.4 or more in the existing method, but the statistics-based stabilization method provided stable results of an ICC of 0.94 or more in all equipment. Through this, when the statistics-based stabilization method is applied, the compatibility between medical image data is secured, and prediction can be stably performed in various environments.

### Distance-based pre-training method

In order to solve the problem that different predicted values occur in the medical images captured under different conditions as described above, the present invention proposes a distance-based pre-training method based on contrastive learning. Through this, the compatibility between medical images is increased, and the performance and stability of the health risk prediction model can be improved.

Contrastive learning is a method of learning the similarity and difference between medical images, and in the medical image data, images of the same patient (that is, the same subject) may be defined as a "positive pair" with each other, and images of a different patient may be defined as a "negative pair." Through this, the feature vectors of various medical images captured under different conditions from the same patient may be learned to become closer.

Whereas, conventionally, contrastive learning learns transformed versions of the same image as a positive pair, in the present invention, a positive pair may be defined at the patient level. Different medical images of the same patient (e.g., the left eye and the right eye, or images with different imaging conditions) are set as a positive pair, so that the health risk prediction model can learn the patient's intrinsic features.

An InfoNCE loss function is used for the contrastive learning, and this may be designed in a manner that maximizes the similarity of the positive pair and increases the distance between the negative pairs. Here, the loss function may be defined as in the following Equation 2. [Equation 2] $\begin{matrix}{L}_{image} \left(y{, y}^{+}\right) = - E \left[log\left(exp\left(sim\left(f\left(y\right),f\left({y}^{+}\right)\right)/τ\right)/Σ_\left\{{y}^{-}∈{N}^{+}\right\}exp\left(\begin{matrix}sim \left(f\left(y\right),f\left({y}^{-}\right)\right) / \\ τ\end{matrix}\right)\right)\right] \\ {L}_{patient} \left(y,y′\right) = - E \left[log\left(exp\left(sim\left(f\left(y\right),f\left(y′\right)\right)/τ\right)/Σ_\left\{{y}^{-}∈N′\right\}exp\left(sim\left(f\left(y\right),f\left({y}^{-}\right)\right)/τ\right)\right)\right] \\ {L}_{total} \left(y{, y}^{+},y′\right) = {L}_{image} \left(y, {y}^{+}\right) + {L}_{patient} \left(y,y′\right)\end{matrix}$

Here, (y, y⁺) is a positive pair at the image level, and (y, y') may mean a positive pair at the patient level.

When the process of selecting a sample from the patient's images and then applying an image augmentation technique is repeated twice, two sets of negative samples are generated, and these may be defined as N⁺ and N', respectively. The set N⁺ is used to calculate the loss of the positive pair at the image level, and the set N' may be used to calculate the loss of the positive pair at the patient level.

According to the above Equation 2, not only the loss at the image level but also the loss at the patient level is reduced together, and accordingly, as the pre-training is performed in a direction of reducing the total loss, the ability of the health risk prediction model to recognize and utilize patient-specific information can be improved, thereby enabling more accurate prediction and analysis.

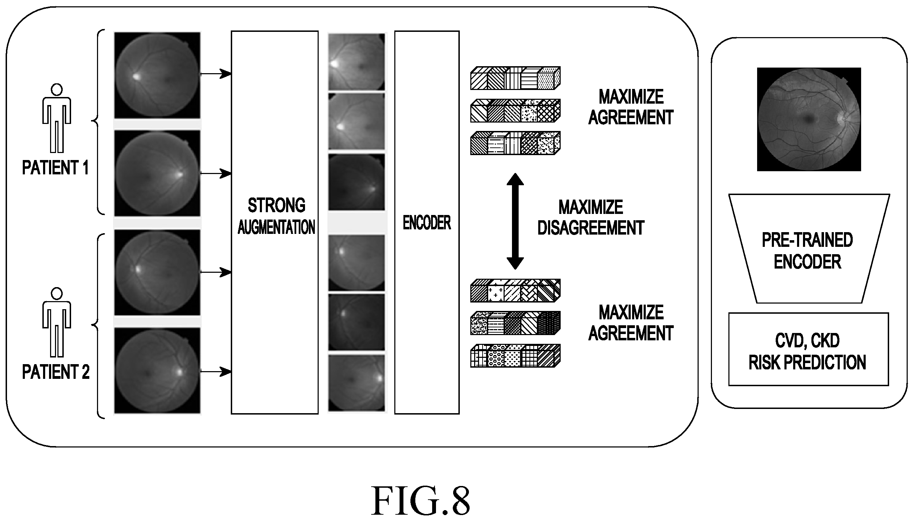

FIG. 8 is a diagram for explaining patient-level contrastive learning according to an embodiment.

Referring to FIG. 8, FIG. 8 visually represents the overall structure of patient-level contrastive learning. The left figure is a contrastive learning framework, in which the training apparatus may set the medical images of the same patient (for example, the left-eye and right-eye images) as a positive pair, set the images of a different patient as a negative pair, and learn the distance between the feature vectors. In this process, the InfoNCE loss function is used, and the loss function may be designed to minimize the distance between the positive pair and maximize the distance between the negative pair. In addition, the right figure shows a structure in which, after the contrastive learning is completed, the health risk prediction model predicts the risk of a specific disease (e.g., cardiovascular disease, kidney disease) through fine-tuning. Through this, the health risk prediction model learns patient-specific features and can provide stable performance even in various medical environments.

More specifically, in FIG. 8, the training apparatus may acquire the left-eye and right-eye images of patient 1 and the left-eye and right-eye images of patient 2. In addition, the training apparatus may perform strong augmentation on the acquired images. In addition, the training apparatus may extract features from the augmented images through an encoder. In addition, the training apparatus may minimize the distance of the positive pair at the patient level by using the InfoNCE loss function. That is, the training apparatus may perform pre-training such that the distance between the feature vectors of the medical images for the same patient is minimized, and perform pre-training such that the distance between the feature vectors of the medical images for different patients is maximized.

The following Table 10 is a table summarizing the results of comparing the performance of a model to which patient-level contrastive learning is applied and the existing learning method. Using AUC (Area Under Curve) and C-Index as performance indicators, the accuracy of the model in cardiovascular disease (CVD) and kidney disease (CKD) prediction is evaluated. In addition, R50 (Resnet-50) and ViT-B (Vision Transformer-Base) were used as architectures.

According to the following Table 10, the image-level contrastive learning showed superior results in all performance indicators compared with the existing method, and in particular, the prediction accuracy was maintained even under various medical data conditions. This shows that patient-level contrastive learning reflects the patient's intrinsic characteristics and contributes to effectively reducing the difference between data. This result proves that the distance-based pre-training method based on patient-level contrastive learning can greatly improve the generalization performance of the health risk prediction model.

**[Table 10]**

| **Method** | **Architecture** | **C-Index** | | **AUC** | |
|---|---|---|---|---|---|
| ImageNet pre-training | R50 | 58.51 | 59.73 | 74.52 | 74.16 |
| Image-level contrastive learning | R50 | 59.29 | 58.98 | 78.07 | 79.67 |
| Patient-level contrastive learning | R50 | **60.85** | **60.79** | **78.88** | **79.79** |
| ImageNet pre-training | ViT-B | 61.16 | 61.23 | 76.97 | 76.92 |
| Image-level contrastive learning | ViT-B | 61.22 | 61.83 | 78.59 | 80.67 |
| Patient-level contrastive learning | ViT-B | **62.6** | **62.99** | **79.26** | **80.85** |

The following Table 11 shows the results of comparing the prediction performance of a model pre-trained with patient-level contrastive learning under various conditions with the existing method. The correlation (Corr) and ICC (Intraclass Correlation Coefficient) were each evaluated according to the conditions of camera type, laterality (left/right eye), and dilation (whether the pupil is dilated).

In the analysis considering the difference in camera type, the model pre-trained with patient-level contrastive learning effectively overcame the data difference between various camera types and recorded the highest correlation and ICC values. In cardiovascular disease (CVD) prediction, the ViT-B-architecture-based patient-level contrastive learning model showed a correlation of 59.78 and an ICC of 52.89, showing performance far superior to the existing method. In kidney disease (CKD) prediction as well, it recorded a correlation of 61.09 and an ICC of 60.68, maintaining consistent performance across various cameras.

In the analysis considering the difference between the left eye and the right eye, the model pre-trained with patient-level contrastive learning recorded a correlation of 81.44 and an ICC of 81.37 in cardiovascular disease (CVD) prediction, showing the highest performance compared with the existing learning method. In kidney disease (CKD) prediction as well, it showed high reliability with a correlation of 73.43 and an ICC of 73.31. This contributes to maintaining data consistency by effectively learning the structural difference between the left and right eyes.

In the analysis considering the difference in whether the pupil is dilated, as a result of analyzing the performance of the model, the model pre-trained with patient-level contrastive learning showed excellent performance in both the pre-dilation and post-dilation data. It maintained high prediction accuracy even in various dilation states, with a correlation of 89.52 and an ICC of 85.72 in cardiovascular disease (CVD) prediction, and a correlation of 84.98 and an ICC of 83.73 in kidney disease (CKD) prediction.

**[Table 11]**

| **Type** | **Method** | **Architect ure** | **Camera type** | | **Laterality** | | **Dilation** | |
|---|---|---|---|---|---|---|---|---|
| | | | **Correlati on** | **ICC** | **Correlati on** | **ICC** | **Correlati on** | **ICC** |
| CVD | ImageNet pre-training | R50 | 44.49 | 35.93 | 71.32 | 71.15 | 82.95 | 76.38 |
| | Image-level contrastive learning | R50 | 32.98 | 15.33 | 77.15 | 76.98 | 89.36 | 85.39 |
| | Patient-level contrastive learning | R50 | **51.94** | **43.06** | **81.44** | **81.37** | **89.57** | **85.72** |
| | ImageNet pre-training | ViT-B | 44.5 | 24.25 | 73.61 | 73.58 | 83.22 | 77.19 |
| | Image-level contrastive learning | ViT-B | 52.48 | 48.86 | 72.32 | 71 | 82.41 | 75.73 |
| | Patient-level contrastive learning | ViT-B | **59.78** | **52.89** | **77.79** | **75.67** | **86.72** | **81.65** |
| CKD | ImageNet pre-training | R50 | 52.43 | 51.51 | 63.14 | 62.87 | 71.42 | 56.62 |
| | Image-level contrastive learning | R50 | 60.86 | 57.79 | 69.15 | 68.2 | 81.24 | 78.42 |
| | Patient-level contrastive learning | R50 | **61.09** | **60.68** | **73.43** | **73.31** | **84.98** | **83.73** |

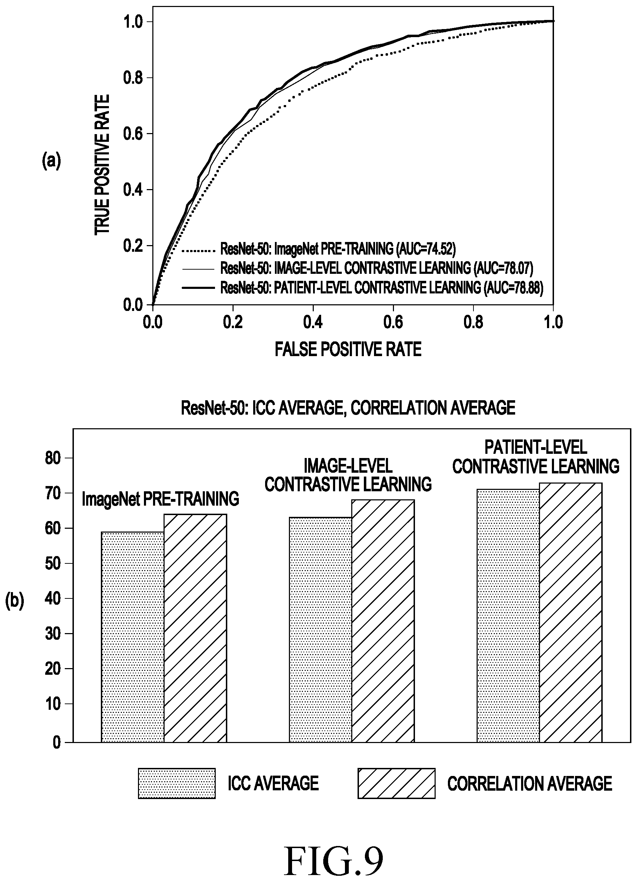

In addition, FIG. 9 is a diagram for explaining prediction performance according to patient-level contrastive learning according to an embodiment.

Referring to FIG. 9, (a) represents the ROC curve of the model to which patient-level contrastive learning is applied and the existing learning method when the architecture is Resnet-50. The X-axis of (a) represents the False Positive Rate, and the Y-axis represents the True Positive Rate. As shown in (a), the AUC of patient-level contrastive learning is shown to be the most superior (AUC = 78.88).

In addition, (b) represents the average ICC and the average correlation of the model to which patient-level contrastive learning is applied and the existing learning method when the architecture is Resnet-50. As shown in (b), the average ICC and the average correlation of patient-level contrastive learning are shown to be the highest.

That is, the contrastive learning technique provides a great advantage in learning features at the image or patient level, and in particular shows higher performance at the patient level.

### Integrated compatibility model

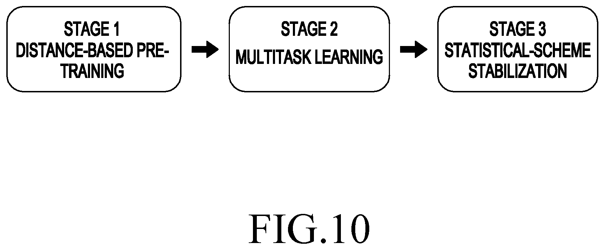

FIG. 10 is a diagram for explaining an integrated compatibility model according to an embodiment.

Referring to FIG. 10, the integrated compatibility model may be modeled in three stages.

First, the training apparatus may perform distance-based pre-training on the health risk prediction model. That is, as a preprocessing process, the training apparatus may perform distance-based pre-training based on contrastive learning. As described above, in order to perform patient-level contrastive learning on the health risk prediction model, the training apparatus may set the medical images of the same patient as a positive pair, set the images of a different patient as a negative pair, and perform pre-training such that the distance between the feature vectors of the positive pairs is minimized and the distance between the feature vectors of the negative pairs is maximized. Through this, the compatibility between medical images is increased, and the performance and stability of the health risk prediction model can be improved.

Second, the training apparatus may perform multitask learning. That is, the training apparatus may perform multitask learning on the first model of the health risk prediction model on which distance-based pre-training has been performed in advance as preprocessing. As described above, the training apparatus may simultaneously learn 21 health indicators together with the fundus photograph on the first model. At this time, the health indicators may be binary-classified based on clinical thresholds, and for example, the training apparatus may simultaneously perform, in a multitask manner, learning of a total of 60 binary classification sub-tasks on the first model. In addition, in this process, the training apparatus may additionally perform self-training. Through this, the first model learns mutually complementary information between the health indicators, thereby providing higher prediction accuracy even on new patient data, improving the generalization performance, and improving the compatibility between medical images.

Third, the training apparatus may perform the statistical-scheme stabilization method. That is, the training apparatus may apply the predicted value output from the first model of the health risk prediction model on which multitask learning has been performed, together with clinical factors such as the patient's age and gender, to the COX-model-based second model, thereby adjusting the prediction result more precisely. Through this, as the second model reflects the clinical meaning, the compatibility between medical images can be improved, and the second model of the health risk prediction model can provide a more sophisticated risk score reflecting the clinical meaning.

The various embodiments of the present specification may be implemented as software including instructions stored in a machine (e.g., computer)-readable storage medium. The machine is a device capable of calling the stored instructions from the storage medium and operating according to the called instructions, and may include an electronic device according to the disclosed embodiments. When the instruction is executed by a processor, the processor may perform the function corresponding to the instruction directly or by using other components under the control of the processor. The instruction may include a code generated or executed by a compiler or an interpreter. The machine-readable storage medium may be provided in the form of a non-transitory storage medium. Here, "non-transitory storage medium" only means that it is tangible and does not include a signal, and does not distinguish whether the data is stored semi-permanently or temporarily in the storage medium. For example, the "non-transitory storage medium" may include a buffer in which the data is temporarily stored.

According to an embodiment, the method according to the various embodiments disclosed in the present specification may be provided by being included in a computer program product. The computer program product may be traded between a seller and a buyer as a commodity. The computer program product may be distributed in the form of a machine-readable storage medium (e.g., Compact Disc Read Only Memory, CD-ROM), or online through an application store (e.g., Play Store^{™}). In the case of online distribution, at least a part of the computer program product (e.g., a downloadable app) may be at least temporarily stored, or temporarily generated, in a storage medium such as a server of a manufacturer, a server of an application store, or a memory of a relay server.

As described above, although the embodiments have been described by the limited embodiments and drawings, those of ordinary skill in the art can make various modifications and variations from the above description. For example, even if the described techniques are performed in an order different from the described method, and/or the components of the described system, structure, apparatus, circuit, and the like are combined or assembled in a form different from the described method, or replaced or substituted by other components or equivalents, appropriate results can be achieved.

Therefore, other implementations, other embodiments, and equivalents to the claims also fall within the scope of the claims described below.

## Claims

1. An apparatus for predicting a health risk based on integrated learning of a plurality of health indicators, for predicting a health risk of a subject, the apparatus comprising:
at least one processor,
wherein the at least one processor is configured to:
input a medical image of the subject to a first model that generates a plurality of predicted health indicators for predicting the health risk of the subject, so as to process the medical image,
selectively extract some health indicators among the plurality of predicted health indicators, and
analyze the health risk of the subject by inputting the selectively extracted health indicators to a second model that predicts the health risk of the subject, so as to process the selectively extracted health indicators,
wherein the first model comprises:
a common sub-model that extracts an overall feature from the medical image to generate a feature vector for one or more tasks; and
one or more head sub-models that receive the feature vector as an input to generate a prediction result for each health indicator,
wherein the first model is trained simultaneously using a medical image and a plurality of health indicators as ground truth labels, and
wherein the plurality of health indicators are defined as labels classified by reflecting respective intrinsic clinical thresholds.

2. The apparatus according to claim 1, wherein the plurality of health indicators are composed of a plurality of binary labels classified by applying the respective intrinsic clinical thresholds.

3. The apparatus according to claim 1 or 2, wherein the first model is subjected to multitask learning for simultaneously predicting the plurality of predicted health indicators.

4. The apparatus according to claim 1, wherein, when a ground truth label for a predetermined health indicator among the plurality of health indicators does not exist at the time of training the first model, a predicted value of the predetermined health indicator is generated through the first model, and the predicted value is used as a predicted ground truth label of the predetermined health indicator so that the first model is additionally trained.

5. The apparatus according to claim 1, wherein the second model is a statistics-based model for analyzing the health risk of the subject based on the plurality of predicted health indicators.

6. The apparatus according to claim 1, wherein the at least one processor is configured to additionally input clinical data of the subject to the second model, and analyze the health risk of the subject by combining the selectively extracted health indicators and the clinical data of the subject.

7. The apparatus according to claim 1, wherein the medical image includes a fundus image.

8. The apparatus according to claim 1, wherein the health risk of the subject includes at least one of cerebro-cardiovascular disease, kidney disease, neurological disease, and liver disease.

9. A method for predicting a health risk based on integrated learning of a plurality of health indicators, for predicting a health risk of a subject, the method comprising:
processing a medical image of the subject by inputting the medical image to a first model that generates a plurality of predicted health indicators for predicting the health risk of the subject; and
analyzing the health risk of the subject by selectively extracting some health indicators among the plurality of predicted health indicators, and inputting the selectively extracted health indicators to a second model that predicts the health risk of the subject so as to process the selectively extracted health indicators,
wherein the first model comprises:
a common sub-model that extracts an overall feature from the medical image to generate a feature vector for one or more tasks; and
one or more head sub-models that receive the feature vector as an input to generate a prediction result for each health indicator,
wherein the first model is trained simultaneously using a medical image and a plurality of health indicators as ground truth labels, and
wherein the plurality of health indicators are defined as labels classified by reflecting respective intrinsic clinical thresholds.

10. The method according to claim 9, wherein the plurality of health indicators are composed of a plurality of binary labels classified by applying the respective intrinsic clinical thresholds.

11. The method according to claim 10, wherein the first model is subjected to multitask learning for simultaneously predicting the plurality of predicted health indicators.

12. The method according to claim 9, wherein, when a ground truth label for a predetermined health indicator among the plurality of health indicators does not exist at the time of training the first model, a predicted value of the predetermined health indicator is generated through the first model, and the predicted value is used as a predicted ground truth label of the predetermined health indicator so that the first model is additionally trained.

13. The method according to claim 9, wherein the second model is a statistics-based model for analyzing the health risk of the subject based on the plurality of predicted health indicators.

14. The method according to claim 9, wherein the analyzing of the health risk of the subject comprises additionally inputting clinical data of the subject to the second model, and analyzing the health risk of the subject by combining the selectively extracted health indicators and the clinical data of the subject.

15. The method according to claim 9, wherein the medical image includes a fundus image.

16. The method according to claim 9, wherein the health risk of the subject includes at least one of cerebro-cardiovascular disease, kidney disease, neurological disease, and liver disease.

17. A recording medium having recorded thereon a computer program for performing any one of the methods of claims 9 to 16.
